(19) Europäisches Patentamt
European Patent Office
Office européen des brevets

(11) Publication number: **0 065 025**
**A1**

# (12) EUROPEAN PATENT APPLICATION

(21) Application number: **81103809.0**

(22) Date of filing: **18.05.81**

(51) Int. Cl.³: **C 07 C 125/06,** B 01 J 23/06, B 01 J 27/04, B 01 J 27/20, B 01 J 31/04, B 01 J 31/06, B 01 J 31/08, B 01 J 31/12, B 01 J 31/22, B 01 J 31/26

(43) Date of publication of application: **24.11.82 Bulletin 82/47**

(84) Designated Contracting States: **BE DE FR GB NL**

(71) Applicant: **THE DOW CHEMICAL COMPANY, Dow Center 2030 Abbott Road Post Office Box 1967, Midland Michigan 48640 (US)**

(72) Inventor: **Gurgiolo, Arthur Emilio, 309 Oak Drive, Lake Jackson Texas 77466 (US)**

(74) Representative: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)**

## (54) Preparation of carbamates from aromatic amines and organic carbonates.

(57) This invention is directed to a process for preparing carbamates from an organic carbonate and an aromatic amine in the presence of a catalyst. The reaction is conducted at a temperature of at least 200 °C and the catalyst is at least one member selected from the group consisting of (1) zinc or divalent tin salts of monocarboxylic acids having a pKa value of less than 2.8 other than such salts of trifluoroacetic acid; (2) divalent cobalt or trivalent cobalt salts of monocarboxylic acids having a pKa value of less that 2.8; (3) zinc or divalent tin or divalent cobalt or trivalent cobalt salts of organic materials having more than one -COOH group per molecule; (4) divalent cobalt or trivalent cobalt salts of a 1,3-diketone having from 5 to 10 carbon atoms; (5) tetravalent alkyl tin compounds or tetravalent alkyl tin oxides represented by formulas I and II:

$$SnR_yX_{4-y} \qquad I$$

and

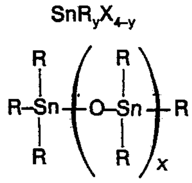

II

wherein each R is independently a hydrocarbon group having from 1 to 10 carbon atoms, or a carboxylate group having from 2 to 10 carbon atoms, X is a halogen atom, x has an average value of from 1 to 5, and y has a value of from 1 to 4; and (6) an inorganic zinc compound selected from zinc carbonate, zinc sulfide, and zinc oxide.

EP 0 065 025 A1

0065025

# PREPARATION OF CARBAMATES FROM AROMATIC AMINES AND ORGANIC CARBONATES

Lewis acids have been disclosed in U.S. 3,763,217 as being suitable catalysts for reacting an organic carbonate with an aromatic amine to prepare carbamates.

It has been unexpectedly discovered that certain zinc, tin and cobalt salts which are relatively inactive as catalysts for promoting the reaction between organic carbonates and aromatic amines at temperatures between 80° and 190°C are active at temperatures of at least 200°C.

The present invention pertains to a process for preparing a carbamate from an organic carbonate and an aromatic amine in the presence of a catalytic quantity of a Lewis acid under suitable reaction conditions to produce said carbamate characterized by conducting the reaction at a temperature of at least 200°C, preferably from 200°C to 300°C, and employing as the Lewis acid at least one member selected from the group consisting of

(1) zinc or divalent tin salts of monocarboxylic acids having a pKa value of less than 2.8 other than such salts of trifluoroacetic acid;

(2) divalent cobalt or trivalent cobalt salts of monocarboxylic acids having a pKa less than 2.8;

(3) zinc or divalent tin or divalent or trivalent cobalt salts of organic materials having more than one -COOH group per molecule;

(4) divalent or trivalent cobalt salts of a 1,3 diketone having from 5 to 10 carbon atoms, preferably from 5 to 6 carbon atoms;

(5) tetravalent alkyl tin compounds or tetravalent alkyl tin oxides represented by formulas I and II

$$SnR_yX_{4-y} \qquad I$$

and

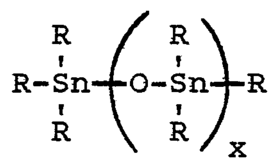

II

wherein each R is independently a hydrocarbon group having from 1 to 10, preferably from 2 to 4 carbon atoms or a carboxylate group having from 2 to 10, preferably from 2 to 4 carbon atoms, X is a halogen atom, preferably chlorine or bromine, x has an average value from 1 to 5, preferably 1 and y has a value of 1 to 4;

(6) an inorganic zinc compound selected from zinc carbonate, zinc sulfide and zinc oxide.

Suitable organic carbonates which can be employed in the process of the present invention include the alkyl, aryl or alkyl aryl esters of carbonic acid. The ester group can be an alkyl group having up to 12 carbon atoms, preferably a lower alkyl group containing up to 6 carbon atoms or the ester group can be an aryl group containing up to 10 carbon atoms.

Particularly suitable organic carbonates are the cyclic and acyclic organic carbonates such as, for example, ethylene carbonate, propylene carbonate, styrene carbonate, dimethyl carbonate, diethyl carbonate, dipropyl carbonate, dibutyl carbonate, dihexyl carbonate, methyl ethyl carbonate, methyl butyl carbonate, diphenyl carbonate, methyl phenyl carbonate, and mixtures thereof.

Any aromatic amine having a conjugate pKa value of from 3.5 to 5.5 is suitable for use herein with those amines having a conjugate pKa value of from 4 to 5.4 being preferred.

Suitable such aromatic amines include those represented by the formulas

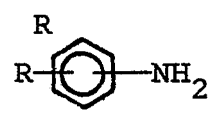

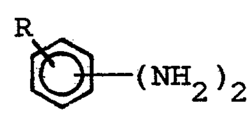

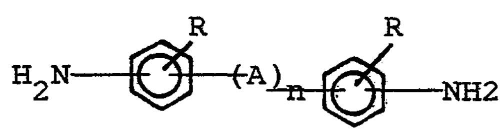

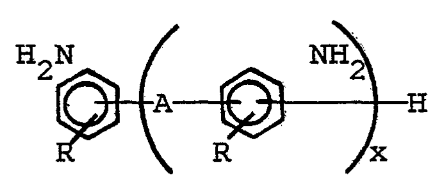

wherein each R is independently hydrogen or a hydrocarbyl or hydrocarbyloxy group containing up to 8 carbon atoms, preferably up to 4 carbon atoms, A is a divalent hydrocarbon group having from 1 to 10 carbon

atoms, preferably from 1 to 6 carbon atoms, n has a value of zero or 1, x has an average value of from 1.1 to 10, preferably from 2 to 4.

Particularly suitable amines include, for example, aniline, o-, m- or p-toluidine, 2,4-xylidine, 3,4-xylidine, 2,5-xylidine, 4-ethylaniline, 3-propyl-aniline, 1,3-diaminobenzene, 2,4-diaminotoluene, 2,6-diaminotoluene, 4,4'-diamino-diphenyl methane, 2,4,4'-triamino-diphenyl ether, 2,6-diamino naphthalene, 4,4'-bis-methylene diphenylamine, o-, m- or p-anisidine, and mixtures thereof.

Suitable zinc or divalent tin or divalent or trivalent cobalt salts of a monocarboxylic acid having a pKa of less than 2.8 which can be employed as a catalyst herein include, for example, zinc dichloro-acetate, zinc trichloroacetate, stannous trichloro-acetate, cobaltous trichloroacetate, cobaltic trichloroacetate, and mixtures thereof. Dichloroacetic acid has a pKa of 1.30 and trichloroacetic acid has a pKa of 0.89.

Suitable zinc or divalent tin or divalent or trivalent cobalt salts of organic materials having more than one -COOH group per molecule which can be employed as a catalyst herein include, for example, zinc oxalate, zinc adipate, zinc citrate, zinc terephthalate, zinc polyacrylate, zinc isophthalate, stannous oxalate, stannous adipate, stannous citrate, stannous terephthalate, stannous polyacrylate, stannous isophthalate, cobaltous oxalate, cobaltous adipate, cobaltous citrate, cobaltous terephthalate, cobaltous polyacrylate, cobaltous isophthalate, cobaltic oxalate, cobaltic

adipate, cobaltic citrate, cobaltic terephthalate, cobaltic polyacrylate, cobaltic isophthalate, and mixtures thereof.

Suitable divalent or trivalent cobalt salts of a 1,3-diketone which can be employed as a catalyst herein include, for example, colbaltous acetylacetonate, cobaltic acetylacetonate, and mixtures thereof.

Suitable tetravalent alkyl tin compounds and tetravalent alkyl tin oxides which can be employed as a catalyst herein include, for example, dibutyl stannic dilaurate, dibutyl stannic maleate, triphenyl stannic acetate, tributyl stannic chloride, dibutyl diphenyl tin hexabutyl ditin, tetraphenyl tin, bis(tributyl tin) oxide, tetrabutyl diacetoxy tin oxide dimer, dibutyl tin oxide polymer, butyl tin trihydroxide, tricyclohexyl tin hydroxide, stannic tetrachloride, and mixtures thereof.

The reaction is generally conducted at a temperature of at least 200°C, preferably from 200°C to 300°C for a time sufficient to complete the reaction depending upon the particular catalyst, reactants and temperature employed. Usually at around 200°C, the time is from 0.25 hour to 8 hours, preferably from 0.5 hour to 2 hours. The reaction generally should be conducted under conditions at which none of the reactants and/or desired product undergo decomposition.

The quantity of catalyst employed usually depends upon the activity of the particular catalyst. Usually from 0.001 mole to 0.2 mole, preferably from 0.005 mole to 0.05 mole of catalyst per mole of the reactant present in a stoichiometric quantity is suitable.

The reactants can be employed on an equimolar basis or one may be present in an excess of the other up to a 20, preferably from one to five moles in excess of the other. It is preferred that the organic carbonate reactant be employed in excess of the aromatic amine.

If desirable and/or convenient the reaction can be conducted in the presence of a solvent. Suitable such solvents include, aromatic hydrocarbons, ethers, and alcohols such as, for example, benzene, toluene, xylenes, diethyl ether, dioxane, ethanol, propanol, methanol, and mixtures thereof.

Uses for the desired carbamate reaction product is suitably discussed by Rosenthal et al in U.S. 3,919,279, U.S. 3,919,280 and U.S. 3,962,302.

The following examples are illustrative of the present invention but are not to be construed as to limiting the scope thereof in any manner.

<u>EXAMPLES 1-26 AND COMPARATIVE RUNS A-O</u>

In the following examples and comparative runs, 5 grams (53.7 mmol) of aniline and 25 grams (277.5 mmol) of dimethyl carbonate were mixed with the amount of catalyst shown in Table I and poured into a stainless steel cylindrical container, 1 5/8" (41.3 mm) internal diameter, and 2 1/4" (57.2 mm) internal depth with a wall thickness of 3/16" (4.8 mm). A stainless steel lid screwed over the reactor with a polytetrafluoroethylene O-ring being used as a seal. Mounted on the lid using stainless steel pipe fittings and parts were a pressure gauge, a pressure relief valve, and a needle valve.

After charging the mixture to the reactor, the lid was sealed by tightening down on the polytetrafluoroethylene O-ring and the reactor was immersed in a hot fluidized sand bath thermostatically controlled at the indicated temperature in the center of the bath. The reactor was heated from between 10 to 15 minutes, then shaken to mix the contents. Then heating was continued for the indicated time. After cooling the contents were analyzed for yields of carbamate and by-products.

The quantity of catalysts, reaction conditions and results are given in the following Table I.

Catalysts employed in Table I and their designations are as follows:

| | |
|---|---|
| Zinc trichloroacetate | A |
| Zinc oxalate | B |
| Zinc citrate | C |
| Zinc adipate | D |
| Zinc terphthalate | E |
| Zinc polyacrylic acid | F |
| Zinc salt of SDVB weak acid cation exchange resin, (13.3% Zn) | G |
| Zinc isophthalate | H |
| Zinc itaconate | I |
| Cobaltous acetylacetonate | J |
| Cobaltic acetylacetonate | K |
| Dibutyl tin dilaurate | L |
| Tetrabutyldiacetoxy tin oxide dimer | M |
| bis(Tributyl tin) oxide | N |
| Triphenyl tin acetate | O |
| Dibutyl tin oxide polymer | P |
| Dibutyl tin maleate | Q |
| Tributyl tin chloride | R |
| Dibutyldiphenyl tin | S |
| Hexabutyl ditin | T |
| Tetraphenyl tin | U |
| Butyl tin trihydroxide | V |
| Tricyclohexyl tin hydroxide | W |
| Zinc carbonate | X |

| | |
|---|---|
| Zinc sulfide | Y |
| Zinc oxide | Z |
| Antimony trichloride | AA |
| Uranium trioxide | BB |
| Uranium dioxide | CC |

In Table I, the pressure listed under Reaction Conditions is guage pressure. Under the heading of Reaction Products, MPC stands for methyl phenyl carbamate, NMA stands for N-methyl anilini, and DPU stands for diphenyl urea. The Mole % Conversion of Anilini to MPC is calculated by dividing the mmols of MPC produced by the mmols of anilini employed (53.7 mmols) and multiplying by 100.

TABLE I

| Example or Comp. Run | Catalyst g/mmol | Reaction Conditions Time Hours | Temp. °C | Pres. psi (MPa) | Reaction Products MPC g/mmol | NMA g/mmol | DPU g/mmol | Mole % Conversion of Aniline to MPC |
|---|---|---|---|---|---|---|---|---|
| A | A/0.97/2.5 | 4 | 140 | 150 (1.03) | 0.3/2 | 0.42/4 | 0.1/0.4 | 3.7 |
| 1 | A/0.97/2.5 | 2 | 200 | 460 (3.17) | 1.4/9.3 | 0.32/3 | 0.04/0.2 | 17.3 |
| B | B/0.947/5 | 4 | 140 | 210 (1.45) | 0/0 | 0/0 | 0/0 | 0 |
| 2 | B/0.47/2.5 | 2 | 200 | 300 (2.07) | 4.82/32 | 15.7/14.7 | 0.09/0.4 | 59.4 |
| C | C/0.508/0.83 | 2 | 140 | 110 (0.76) | 0.06/0.4 | 0.44/4.1 | 0.02/0.1 | 0.7 |
| 3 | C/0.511/0.84 | 2 | 200 | 350 (2.41) | 3.34/22 | 2.67/25 | 0/0 | 41.1 |
| D | D/0.524/2.5 | 2 | 140 | 150 (1.03) | 0/0 | 0.2/1.6 | 0.0 | 0 |

TABLE I Con'd

| Example or Comp. Run | Catalyst g/mmol | Reaction Conditions: Time Hours | Temp. °C | Pres. psi (MPa) | Reaction Products: MPC g/mmol | NMA g/mmol | DPU g/mmol | Mole % Conversion of Aniline to MPC |
|---|---|---|---|---|---|---|---|---|
| 4 | D/0.53/2.5 | 1 | 200 | 220 (1.52) | 0.77/5 | 0.8/7.5 | 0.44/2 | 9.5 |
| E | E/0.58/2.5 | 2 | 140 | 160 (1.10) | 0.03/0.2 | 0.3/2.7 | 0/0 | 0.4 |
| 5 | E/0.58/2.5 | 2 | 200 | 390 (2.69) | 3.62/24 | 2.38/22 | 0.04/0.2 | 44.6 |
| F | F/5.0/2.5 | 3.5 | 160 | 200 (1.38) | 0.06/0.4 | 0.7/6.5 | 0.08/0.4 | 0.7 |
| 6 | F/5.0/2.5 | 2 | 200 | 600 (4.14) | 7.07/47 | 0.78/7.3 | 0.06/0.3 | 87.1 |
| G | G/2.66/40 | 4 | 140 | 200 (1.38) | 0/0 | 0/0 | 0/0 | 0 |
| 7 | G/2.66/40 | 2 | 200 | 390 (2.69) | 2.12/14 | 2.88/23 | 0.012/0.1 | 26.1 |
| H | H/0.580/2.5 | 2 | 140 | 160 (1.10) | 0.13/0.9 | 0.32/3 | 0.02/0.1 | 1.7 |

TABLE I Con'd

| Example or Comp. Run | Catalyst g/mmol | Reaction Conditions: Time Hours | Reaction Conditions: Temp. °C | Reaction Conditions: Pres. psi (MPa) | Reaction Products: MPC g/mmol | Reaction Products: NMA g/mmol | Reaction Products: DPU g/mmol | Mole % Conversion of Aniline to MPC |
|---|---|---|---|---|---|---|---|---|
| 8 | H/0.580/2.5 | 2 | 200 | 750 (5.17) | 4.4/29 | 1.1/10 | 0.07/0.3 | 54.0 |
| I | I/0.484/2.5 | 7 | 140 | 150 (1.03) | 0.5/3.3 | 0.65/6 | 0/0 | 6.1 |
| 9 | I/0.484/2.5 | 2 | 200 | 350 (2.41) | 2.3/15 | 1.14/11 | 0.16/0.8 | 27.9 |
| 10 | J/0.643/2.5 | 2 | 200 | 200 (1.38) | 5.95/39.3 | 0.75/7 | 0.03/0.13 | 73.2 |
| 11 | K/0.891/2.5 | 2 | 200 | 200 (1.38) | 5.48/36.3 | 0.87/8 | 0.125/0.6 | 67.5 |
| J | L/1.58/2.5 | 1 | 140 | 120 (0.83) | 0.31/2 | 0.17/1.6 | 0.13/0.6 | 3.8 |
| 12 | L/1.58/2.5 | 1 | 200 | 240 (1.65) | 7.1/47 | 0.16/1.5 | 0.13/0.6 | 87.5 |
| K | M/3.0/5 | 2.5 | 140 | 150 (1.03) | 3.6/24 | 0.18/1.7 | 0.28/13 | 44.2 |

TABLE I Con'd

| Example or Comp. Run | Catalyst g/mmol | Reaction Conditions: Time Hours | Temp. °C | Pres. psi (MPa) | Reaction Products: MPC g/mmol | NMA g/mmol | DPU g/mmol | Mole % Conversion of Aniline to MPC |
|---|---|---|---|---|---|---|---|---|
| 13 | M/0.75/1.25 | 1 | 200 | 210 (1.45) | 7.41/49 | 0.33/3.1 | 0.13/0.6 | 91.3 |
| 14 | N/0.75/2.5 | 1 | 200 | 210 (1.45) | 0.84/5.6 | 0.7/6.5 | 0.12/0.56 | 10.4 |
| 15 | O/1.023/2.5 | 1 | 200 | 240 (1.65) | 4.16/27.5 | 0.51/4.8 | 0.13/0.6 | 51.3 |
| 16 | P/0.622/2.5 | 1 | 200 | 220 (1.52) | 7.11/47 | 0.17/1.6 | 0.06/0.3 | 87.6 |
| 17 | Q/0.873/2.5 | 1 | 200 | 280 (1.93) | 5.27/35 | 0.23/2.2 | 0.16/0.75 | 65.0 |
| 18 | R/0.814/2.5 | 1 | 200 | 300 (2.07) | 0.90/6 | 1.25/12 | 0.16/0.75 | 10.8 |
| 19 | S/0.97/2.5 | 4 | 200 | 310 (2.14) | 0.05/0.3 | 0.75/7 | 0.23/1 | 0.6 |
| 20 | T/0.725/2.5 | 1 | 200 | 300 (2.07) | 0.47/3.1 | 0.72/6.7 | 0.17/0.8 | 5.8 |

TABLE I Con'd

| Example or Comp. Run | Catalyst g/mmol | Reaction Conditions Time Hours | Temp. °C | Pres. psi (MPa) | Reaction Products MPC g/mmol | NMA g/mmol | DPU g/mmol | Mole % Conversion of Aniline to MPC |
|---|---|---|---|---|---|---|---|---|
| 21 | U/1.068/2.5 | 1 | 200 | 310 (2.14) | 2.82/18.7 | 0.7/6.3 | 0.14/0.7 | 34.7 |
| 22 | V/0.567/2.5 | 1 | 200 | 300 (2.07) | 6.15/41 | 0.37/3.4 | 0.10/0.5 | 75.6 |
| 23 | W/0.525/2.5 | 1 | 200 | 280 (1.93) | 0.71/4.7 | 0.76/7.1 | 0.18/0.8 | 8.8 |
| 24 | X/0.314/2.5 | 2 | 200 | 260 (2.48) | 7.5/50 | 0.23/2.2 | 0.05/0.22 | 92.4 |
| L | X/0.314/2.5 | 0.5 | 190 | 190 (1.31) | 0.33/2.2 | 0.55/5 | 0.14/0.7 | 4.1 |
| 25 | Y/0.407/5 | 2 | 200 | 390 (2.69) | 3.03/20 | 2.8/26 | 0.07/0.33 | 37.3 |
| 26 | Z/1.0/10 | 2 | 200 | 390 (2.69) | 3.4/22.4 | 2.6/24 | 0.04/0.17 | 41.8 |
| M | AA/0.57/2.5 | 2 | 200 | 280 (1.93) | 1.7/11 | 0.85/8 | 0.06/0.26 | 21.2 |

TABLE I Con'd

| Example or Comp. Run | Catalyst g/mmol | Reaction Conditions Time Hours | Temp. °C | Pres. psi (MPa) | Reaction Products MPC g/mmol | NMA g/mmol | DPU g/mmol | Mole % Conversion of Aniline to MPC |
|---|---|---|---|---|---|---|---|---|
| N | BB/1.602/5.6 | 2 | 200 | 700 (4.83) | 4.464/29.5 | 6.37/60 | 0.67/3 | 14.8 |
| O | CC/1.512/5.6 | 2 | 200 | 650 (4.48) | 4.544/30.1 | 6.35/60 | 0.64/3 | 15.0 |

# CLAIMS

1. A process for preparing carbamates from an organic carbonate and an aromatic amine in the presence of catalytic quantities of a catalyst characterized by conducting the reaction at a temperature of at least 200°C and employing as the catalyst at least one member selected from the group consisting of

(1) zinc or divalent tin salts of monocarboxylic acids having a pKa value of less than 2.8 other than such salts of trifluoroacetic acid;

(2) divalent cobalt or trivalent cobalt salts of monocarboxylic acids having a pKa value less than 2.8;

(3) zinc or divalent tin or divalent or trivalent cobalt salts of organic materials having more than one -COOH group per molecule;

(4) divalent cobalt or trivalent cobalt salts of a 1,3 diketone having from 5 to 10 carbon atoms;

(5) tetravalent alkyl tin compounds or tetravalent alkyl tin oxides represented by formulas I and II

$$SnR_yX_{4-y} \qquad \text{I}$$

and

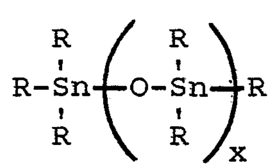

II

wherein each R is independently a hydrocarbon group having from 1 to 10 carbon atoms, or a carboxylate group having from 2 to 10 carbon atoms, X is a halogen atom, x has an average value from 1 to 5, and y has a value of 1 to 4; and

(6) an inorganic zinc compound selected from zinc carbonate, zinc sulfide and zinc oxide.

2. The process of Claim 1 wherein the reaction temperature is from 200°C to 300°C.

3. The process of Claims 1 or 2 wherein said catalyst is zinc carbonate, the zinc salt of an acid containing ion exchange resin, zinc salt of polyacrylic acid, cobaltous acetylacetonate, or mixtures thereof.

European Patent Office

**EUROPEAN SEARCH REPORT**

0065025

Application number

EP 81103809.0

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| D,X | US - A - 3 763 217 (BRILL)<br>* Column 3, lines 22, 59-69; claims 1-3 *<br>---- | 1,2 | C 07 C 125/06<br>B 01 J 23/06<br>B 01 J 27/04<br>B 01 J 27/20<br>B 01 J 31/04<br>B 01 J 31/06<br>B 01 J 31/08<br>B 01 J 31/12<br>B 01 J 31/22<br>B 01 J 31/26 |
| | | | TECHNICAL FIELDS SEARCHED (Int. Cl.³) |
| | | | C 07 C 125/00<br>B 01 J |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| VIENNA | 11-12-1981 | HERING |

EPO Form 1503.1 06.78